Europäisches Patentamt

European Patent Office

Office européen des brevets

(11) Publication number: **0 437 636 A1**

# EUROPEAN PATENT APPLICATION
## published in accordance with Art. 158(3) EPC

(21) Application number: 90912887.8

(22) Date of filing: 06.07.90

(86) International application number:
**PCT/SU90/00177**

(87) International publication number:
**WO 91/01709 (21.02.91 91/05)**

(51) Int. Cl.⁵: **A61H 39/00, A61N 5/06**

(30) Priority: 07.08.89 SU 4728030

(43) Date of publication of application:
**24.07.91 Bulletin 91/30**

(84) Designated Contracting States:
**CH DE FR GB IT LI**

(71) Applicant: **NAUCHNO-ISSLEDOVATELSKY INSTITUT RADIOPRIBOROSTROENIA
Leningradsky pr., 80
Moscow, 125178(SU)**

(72) Inventor: **ZHUKOV, Dmitry Sergeevich
ul. 800-letia Moskvy, 5-3-221
Moscow, 127591(SU)**
Inventor: **KHVATOV, Leonid Georgievich
Kutuzovsky pr., 4/2-270
Moscow, 121248(SU)**
Inventor: **RYCHKOV, Jury Viktorovich
ul. Kulakova, 12-1-402
Moscow, 123592(SU)**
Inventor: **GAPONJUK, Petr Yakovlevich
ul. Osennaya, 2-12
Moscow, 121357(SU)**

(74) Representative: **Waldren, Robin Michael et al
Marks & Clerk, 57-60 Lincoln's Inn Fields
London WC2A 3LS(GB)**

(54) DEVICE FOR LOCATING AND INFLUENCING ACUPUNCTURE POINTS.

(57) The device comprises an active electrode (8) containing a heat radiator (2) with a reflector (1) and a light guide (4) of a passive electrode (13), as well as a control system (3). The heat radiator (2) with the reflector (1) is connected to the control system (3), whereas the light guide (4) consisting of a focusing system is located on the same optical axis (OO') as the reflector. The larger base of the focusing system (4) faces the heat radiator (2), whereas the smaller one is intended for contact with the the skin surface (7) due to which the optical system has a double focusing capacity and allows to concentrate the needed radiation power at the output of the smaller base (6) of the focusing system (4).

## TECHNICAL FIELD

The invention relates to the medicine, and in particular, to the reflexotherapy and may be used for the prophylaxis of various dieseases as well as for treatment of rhinitis, bronchitis, bronchial asthma, neuralgia, insomnia, arthritis, vegetovascular dystonia, headche, colitis, impotence, and also in sport medicine.

Background Art

Side effects such as allergia, weakening of immunological response of the body, suppression of endocrine organs, and the like become more frequent nowadays when medical sulbstances are used for a prolonged period.

Reflexotherapy is one of powerful instruments enhancing functional capabilities of the body and is effectively used for controlling malfunction of individual systems or organs.

There are a large number of methods of stimulating acupuncture points, and most popular among them are acupuncture and cauterisation with herbaceous cigar. Most frequently usded in the medical practice is the acupuncture, and cauterisation with herbaceous cigar is used rather rarely because of a shortage of herbaceous cigars and inconvenience of this technique, in particular because of smoke.

However, the acupuncture is the invasive method which calls for the introduction of a needle into a human body which is undesirable bacause of painfulness of this procedure and also because of the danger of conracting hepatitis, AIDS and other infectious diseases.

For this reason, during recent three decades, devices have come into use in the medical practice which are capable of simulating the exposure of the body to traditional actions by means of certain physical stimulation of acupuncture points.

Non-invasive methods allowing acupuncture points to be stimulated by electricity, coherent (non-coherent) light and heat have come into a widespread use.

Laseropuncture involving the exposure of acupuncture points to monochrome coherent radiation is now widely used. For that purpose, use is made of low-power lasers generating radiation in the red area of the optical range. However, laser is an expensive apparatus and requires special operating conditions. In addition, studies have shown that the action of coherent light upon acupuncture points does not have the specific effect in comparison with non-coherent white light of the same intensity; moreover, non-linear properties of the skin result in the propagation of white light occurring with a lower attenuation which is likely to be linked with the biological evolution of living body.

Another kind of reflexotherapy is the electropuncture or electrical suface stimulation of acupuncture points.

Known in the art are a number of apparatuses for locating acupuncture points and stimulating them, e.g., an electronic acupuncture instrument (DE, A, 3631600).

The electronic acupuncture instrument consists of a metal casing which houses a power supply and an electronic circuit connected to a stimulating metal electrode (active electrode). The instrument casing functions as passive electrode. The instrument is capable of locating acupuncture points on the skin and activating them in accordance with the necessary treatment procedure by varying polarity and intensity of current. The location is determined by pressing the active electrode against the skin in the area of the selected acupuncture point and by moving the electrode. When the point is located, current flowing in the circuit active electrode - skin - passive electrode increases, and a threshold member operated to initiate a sound alarm signal. The point is exactly located by the sound pitch.

The instrument simulates the acupuncture, but it cannot simulate cauterisation of acupuncture points.

Also known in the art is an apparatus for radio-frequency exposure of skin areas with the aim of cauterizing (FR, A, 2608917).

This apparatus allows simultaneous location of acupuncture points and cauterisation of a predetermined acupuncture point by means of an active electrode.

The apparatus combines a device for locating acupuncture points, a device for stimulating them, and a power supply in the form of a rechargeable storage battery in a common casing. In addition to the casing, the apparatus has active and passive electrodes. The passive electrode is in the form of a plate applicable to any zone of the skin and the active electrode is in the form of a narrow feeler (probe).

Acupuncture points are located by means of bipolar rectangular pulses. With the passive electrode applied to the skin, movement of the active electrode in the area of the acupuncture point results in an increase in the current flowing in the circuit passive electrode - skin - active electrode. In case a threshold level is overpassed, an alarm signal is generated. Subsequently the acupuncture point is exposed to high-frequency current with the aim of cauterizing it.

It should be noted that cauterization (heating) of the skin with high-frequency current is radically different from heating of the skin, e.g., by a radiation in the optical range. The biological effect of the energy in the high-frequency radiorange does not simulate the effect of cauterization with herbaceous cigar which ensures radiation in the near and medium infrared range. In addition, the radio-frequency cauterization has not been studied enough and can have undesirable side effects.

The most similar to the invention is an apparatus disclosed in FR, A, 2358279.

This apparatus is based on luminous and thermal radiation which is concentrated at acupuncture points.

The apparatus comprises a thermal radiator in the form of an electric lamp having, on one side, a parabolic reflector and on the other side, an attachment functioning as a light guide. A metal attachment having its inner surface in the form of frustoconical surface has an opening aligned with the optical axis of the reflector.

The thermal radiator is at the focus of the parabolic reflector. When the electric lamp is heated, the reflected thermal and luminous energy propagates through the attachment and concentrates at the acupuncture points.

However, the focusing conditions are such that it is very difficult to achieve sufficient temperature on the skin surface because of the losses in the optical device. To obtain high optical power, a strong heating of the electric lamp filament is required. In such case the spectrum of the luminous radiation is shifted towards the lower boundary of the light range. This results in differences between spectrum of wormwood cigar and emission spectrum acting upon the acupuncture point as provided by this apparatus. Therefore, the effect of wormwood cigar on acupuncture points cannot be achieved.

Disclosure of the Invention

It is an object of the invention to provide an apparatus for locating and activating acupuncture points which, owing to the construction of an active electrode, allows exact concentration of optical energy with spectral characteristics corresponding to the spectrum of herbaceous cigars to be achieved at the located acupuncture point.

This object is accomplished by that in an apparatus comprising a thermal radiator having a reflector and connected to a control circuit and a light guide aligned with the optical axis of the reflector, according to the invention, the light guide is in the form of a focon (focusing element) having its larger base facing towards the thermal radiator and psotioned in the focal plane of the reflector and its smaller base in contact with the skin.

This allows a substantially high temperature in accordance with medical prescriptions to be achieved on the skin by using an optical radiation in a predetermined spectral band. This ensures concentration of the luminous and thermal action upon acupuncture points.

The inner surface of the reflector may be in the form of an ellipsoid of revolution, the larger base of the focon being positioned at the second focus of the reflector.

This allows high efficiency of the optical part of the apparatus to be ensured by lowering losses of energy supplied from the thermal radiator to an acupuncture point.

The diameter of the larger base of the focon preferably corresponds to the outlet diameter of the reflector, the diameter of the smaller base corresponding to the width of the optical directional pattern of an acupuncture point.

This ensures best conditions for focusing, hence, best conditions for concentrating energy within a predetermined space.

An electrically conductive coating may be provided on at least a part of the outer surface of the focon. The coating thickness, when added to the diameter of the smaller base or the focon, corresponds to the width of the directional pattern of electrical conductance of the acupuncture point, the coating being connected to a control circuit. This facility allows the apparatus to be used for location of acupuncture points and for their stimulatin with electric current.

It is preferred that the outside diameter of the smaller base of the focon, which is in contact with the skin, correspond to an averaged size of an acupuncture point. This allows the space in which energy is to be concentrated to correspond to the typical size of acupuncture points.

The control circuit may have a power supply connected, via a current regulator, to the thermal radiator. This facility allows temperature on the skin to be controlled and intensity of optical radiation to be corrected.

The apparatus may have the passive electrode on the skin and a pulse generator having an input to which the passive electrode is connected, the output of the generator being connected, via a current regulation, to the electrically conductive coating. This allows a closed electric circuit passive electrode - skin - active electrode to be made and ensures the exposure of acupuncture points to electric current.

The apparatus may have a threshold circuit with a sound alarm system connected, via a switch, to the output of the pulse generator. This allows location of acupuncture points having a conductance which is higher than that on the rest of the skin to be carried out in this circuit using as criterion an increase in electric current in excess of a threshold value.

The passive electrode may be in the form of an adjustable diameter envelope which can be put on a finger. This allows, in using the medical procedure of use of the apparatus, depending on the disease, the passive electrode to be put on a certain finger so as to enhance efficiency of the apparatus in use.

Brief Description of the Drawings

The invention will now be described in detail with reference to specific embodiments illustrated in the accompanying drawings, in which:

Fig. 1 is a general view of an apparatus for locating and stimulating acupuncture points with a control circuit according to the invention;

Fig. 2 is an illustration of construction of a focon (longitudinal section) according to the invention;

Fig. 3 is an optical directional pattern of an acupuncture point according to the invention;

Fig. 4 is an electrical conductunce directional pattern of acupuncture points according to the invention.

Best Way of Carrying out the Invention

An papparatus according to the invention has a reflector 1 having a thermal radiator 2 positioned in its focus and connected to a control circuit 3, and a light guide in the form of focon 4.

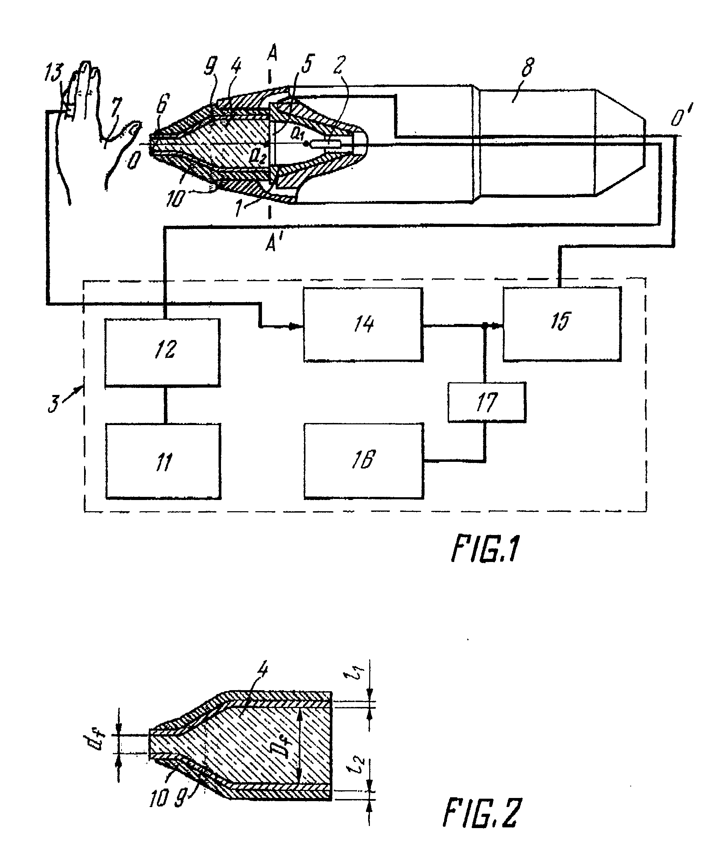

A larger base 5 of focon 4 faces towards thermal radiator 2 and its smaller base 6 is designed to be brought in contact with skin 7 of a body. Focon 4 according to the invention may be made of glass, or it may be hollow and of metal. Focon 4, which is capable of providing internal reflection, allows an additional focusing of luminous energy to be ensured with a gain which depends on the ratio of diameters $D_f$ and $d_f$ of focon 4 (Fig. 2).

Thermal radiator 2 of the apparatus according to the invention is in the form of a halogen lamp. By varying supply voltage of the halogen lamp, i.e., incadescence temperature of its filament, an emission spectrum can be chosen, on the basis of the Max Planck law, which will be the closest one to the spectrum of wormwood cigar. If the supply voltage decreases, the emission spectrum is shifted to the red and with a voltage increase, it is shifted to the violet. Thermal radiator 2 may be in the form of any other noncoherent radiation source which has a small enough size in comparison with the aperture of reflector 1.

The inner surface of reflector 1 according to the invention may be in the form of an ellipsoid of revolution, the halogen lamp being positioned in the first focus $a_1$ of the ellipsoid and larger base 5 of focon 4 being disposed in a focal plane AA' of reflector 1. With this position of various components, the optical system has a double focusing capacity which allows energy of radiation to be concentrated in an optimum manner so as to ensure the desired radiation power at the outlet of smaller base 6 of focon 4.

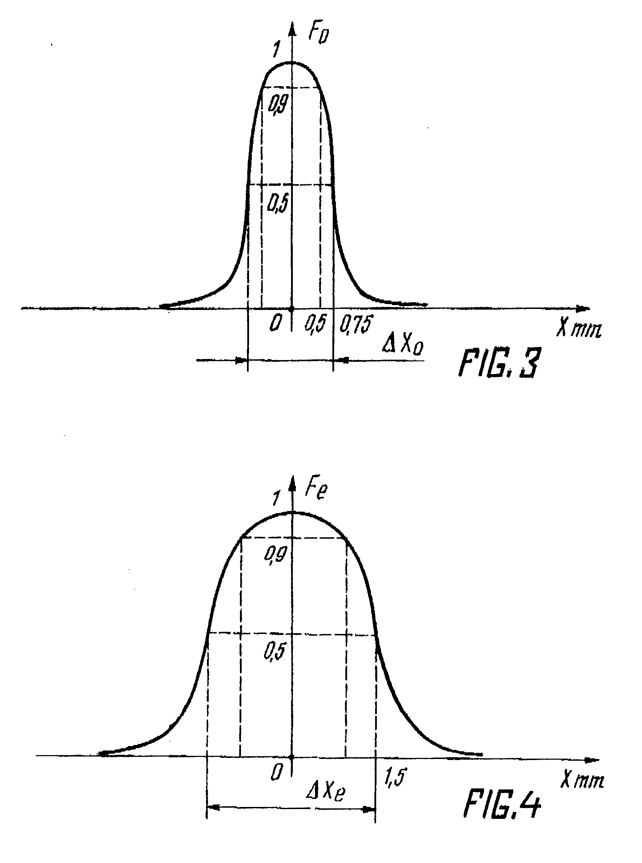

Diameter $D_f$ of larger base 5 of focon 4 according to the invention may correspond to the aperture size of reflector 1 and the diameter $d_f$ of smaller base 6 may correspond to the width of the optical directional pattern (Fig. 3) which characterizes reception of light radiation by an acupuncture point at the level of one half of maximum received power of optical radiation.

Since lamp 2 is a finite source of radiation and cannot be regarded as a point-like source, its image in the focal plane AA' will be in the form of a blurred sport with dimensions which are greater than those of the filament of halogen lamp 2. The use of focon 4 allows an additional focusing of the focal spot to be ensured thereby enhancing power at larger base 6 of focon 4. With short-focus reflectors 1 used for the apparatus according to the invention to ensure small size of the active electrode 8, the dimentions of the focal spot and basic side spots are such as to concentrate them in a space defined by the aperture of the reflector in a plane drawn perpendicularly with respect to the optical axis. In this connection, for full utilization of luminous and thermal energy, diameter $D_f$ of larger base 5 of focon 4 is equal to the aperture of reflector 1.

Fig. 3 shows an optical directional pattern of an acupuncture point $F_o(x)$ which is in the form of a gra - phical image of an optical radiation penetrating acupuncture points depending on the distance $\Delta x$ from the active electrode to the acupuncture point. If the electrode is shifted at a distance of $\Delta x_o = 0.75$ mm in any direction from the center of an acupuncture point, power of penetrating radiation becomes two times smaller or 3 Dd lower. The width of the directional pattern determined by the level 3 Dd is the conventional characteristic for aerial devices. For this reason, diameter $d_f$ of smaller base 6 of focon 4 is chosen to be equal to the width of the optical directional pattern of the acupuncture points. In thes case, the size of smaller base 6 of focon 4 is about 1.5 mm.

The outer periphery of focon 4 may have an electrically conductive coating 9. Fig. 4 is a diagram of electrical conductance of an acupuncture point $F_e(x)$ which is in the form of a graphic representation of electric current flowing through an acupuncture point vs. the distance $\Delta x$ from the center of the acupuncture point. It can be seen from this diagram that if the distance $\Delta x_e = 1.5$ mm, conductance in the zone of the point is twice as low as that at the center of the point. The width of the diagram of electrical concuctance of an acupuncture point at the level of $1/2\ F_e(O)$ characterizes the preferred size of the electrode for acting with electric current, i.e., the maximum diameter of 3 mm.

Bearing in mind that the location and stimulation are carried out with electric pulses with a broad enough frequency spectrum and that the skin-effect in this case results in the electric pulse propagating through the periphery of nervous fibers, electric pulses can be supplied by means of an annular electrode deposited on top of focon 4 rather than by means of a continouus electrode. The best matching of the electrode to the skin at the acupuncture point in this case is achieved if the outside diameter of the metal ring of the electrically conductive coating is equal to the width of the diagram of electric conductance of the acupuncture point. Since on electrically conditive cating 9 is deposited on focon 4, and an insulating coating 10 can also be provided, the outside diameter of focon 4 means the total diameter taking into account eventual coatings which are in contact with the body skin. The outside diameter of focon is limited by the averaged size of an acupuncture point and is of the order of 5 mm.

The apparatus may have control circuit 3 which comprises a power supply 11 connected, via a current regulator 12, to thermal radiator 2. This allows only luminous and thermal action to be exerted upon acupuncture points with a controlled intensity of luminous flux and temperature of the skin 7.

The apparatus may have a passive electrode 13 in the form of an envelope which can be easily applied to the skin 7 during operation by putting it on any finger of a patient. Passive electrode 13 is connected to a pulse generator 14 having its input connected via a current regulator 15, to electrically conductive coating 9. The apparatus may have a thershold circuit 16 with a sound alarm system which is connected, via a switch 17, to the output of pulse generator 14.

When active electrode 8 falls on an acupuncture point which has higher conductance, a closed electric circuit passive electrode - acupuncture channel - active electrode is made in which a current in excess of the threshold current flows, and the sound alarm system will operate. The points are scanned by short bipolar pulses and are located by the intensity of the sound alarm signal.

The apparatus functions in the following manner. The apparatus is plugged in by means of a power supply cord, an internal general power toggle switch is turned on, and an indicator lamp glows on the control board of the instrument. Voltage is thus applied to power supply 11 of thermal radiator 2, pulse generator 14, and threshold circuit 16 with a sound alarm system.

With the use of a diagram showing location of acupuncture points on skin 7 and in accordance with medical prescriptions,a necessary point on skin 7 is found and located. Regulator 12 is then used to set up the desired power of optical radiation to produce a high temperatur on skin 7. A pricking or ironing action is then exerted by touching skin 7 with active electrode 8.

If it is desired to act with electric current, passive electrode 13 is put on a finger, and regulator 15 is operated to set up the necessary current value. The current value for the exposure is set up using mainly three control ranges: for the face, trunk, and feet.

For scanning acupuncture points and locating them, the passive electrode is put on a finger, and threshold circuit 16 is turned on by setting switch 17 to the mode of location of acupuncture points. Active electrode 8 is applied to skin 7,and electric current flows in the circuit formed by electrically conductive surface 9 - skin7-- passive electrode 13 - pulse generator 14 - switch 17 - threshold circuit 16 - regulator 15, the value of the current being determined by conductance of skin 7 and setting of regulator 15. Regulator 15 is operated to set up a threshold sensitivity of the skin in the vicinity of an acupuncture point by determining the minimum level of sound alarm signal. The regulator 15 is then turned in the oppositve direction until the sound alarm signal disappears. The acupuncture point is then located by moving active electrode 8 positioned perpendicularly with respect to skin 7, and this is determined by the sound alarm signal. By moving active electrode 8 slowly with one and the same pressure force the acupuncture point is located more accurately by achieving the maximum sound level. The point is marked on skin 7 by mechanically pressing active electrode 8. The location device is then turned off. Scale divisions of regulator 12 are used to make choice, in accordance with the medical instructions, of the necessary optical power so as to produce the desired temperature on skin 7. The stimulation procedure is then started by exerting pricking or ironing action in accordance with the desired result - suppressing or stimulating effect on the patient.

If it is desired to expose an acupuncture point to electric current, current amplitude is chosen by turning regulator 15, and the exposure prodecure is then carried out. Focon 4 having continoous insulating coating 10 converging towards smaller base 6 of focon 4, in addition to the electrical, luminous and thermal action, allows the mechanical action such as massage to be carried out as well, without injuries to the peripheral zone of the acupuncture point.

To promote the effect of stimulating of acupuncture points, all kinds of action can be used simulteneously.

Simulteneously using luminous and thermal action and electric current, due to heating of the skin and secretion of intercellular liquid during heating, most favourable conditions of exposure to electric current are created owing to that effectiveness of the procedure increases.

Industrial Applicability

The apparatus according to the invention allows corporal and auricular acupuncture points to be located with enhanced accuracy, and highly efficient contact luminous and thermal action such as cauterization can be effected in combination with electrical stimulation and massage. Location of points is accompanied by sound alarm signals.

The invention may be used for facusing luminous and thermal flux with a high degree of accuracy within a small area which is commensurable with an acupuncture point thereby ensuring a substantially high density of power of optical flux.

The advantage of the invention resides in the possibility of use of a noncoherent light source and of exerting an action upon acupuncture points with waves over the whole visible spectral range (white light) which can better penetrate the body through acupuncture channels in comparison with laser radiation.

The spectrum at the outlet of the focon can correspond to the spectrum of cigars prepared of herbs, such as wormwood used in the oriental medicine.

The invention can be advantageously used for the prophylaxis and treatment of respiratory infections, pathologies of internal organs, neurological, gynecological and skin diseases, impotence, locomotor disorders, and somking.

With high efficiencey, the apparatus according to the invention is substantially simpler and cheaper than laser apparatus while retaining all capabilities of electrical stimulation and cauterization devices used for acupuncture points.

**Claims**

1. An apparatus for locating and stimulating acupuncture points on the skin (7) of a body, comprising a thermal radiator (2) having a reflector (1) and connected to a control circuit (3), and a light guide (4) aligned with the optical axis (OO') of the reflector (1), **characterized** by that the light guide (4) is in the form of a focon having its larger base (5) facing towards the thermal radiator (2) and its smaller base (6) in contact with the skin (7).

2. An apparatus according to claim 1, **characterized** in that the larger base (5) of the focon is in the focal plane AA' of the reflector (1).

3. An apparatus according to claim 1, **characterized** in that the inner surface of the reflector (1) is in the form of an ellipsoid of revolution having its first focus $a_1$ at which the thermal radiator (2) is positioned, the larger base (5) of the focon (4) being positioned at the second focus $a_2$.

4. An apparatus according to claim 1, **characterized** in that the diameter of the larger base (5) of the focon corresponds to the outlet diameter of the reflector (1), the diameter of the smaller base (6) corresponding to the width of the optical directional pattern (Fig. 3) of an acupuncture point.

5. An apparatus according to claim 1, **characterized** in that, for location and stimulation, at least a part of the periphery of the focon (4) is externally provided with an electrically conductive coating (9) having a thickness $(l_1)$ which, when added to the diameter of the smaller base (6) of the focon (4), corresponds to the width of the directional pattern of electric conductance (Fig. 4) of an acupuncture point, the coating (9) being connected to the control circuit (3).

6. An apparatus according to claim 5, **characterized** in that the outside diameter of the smaller base (6) of the focon (4) which is in contact with the skin (7) corresponds to the averaged size of an acupuncture point.

7. An apparatus according to claim 1, **characterized** in that the control circuit (3) comprises a power supply (11) connected, via a current regulator (12), to the thermal radiator (2).

8. An apparatus according to claim 1, **characterized** in that it comprises a passive electrode (13) operatively connected to the skin (7) and a pulse generator (14) having an input to which is connected to the passive electrode (13) and an output of the generator (14) is connected via a current regulator (15), to the electrically conductive coating (9).

9. An apparatus according to claim 8, **characterized** in that it comprises a threshold circuit (16) with a sound alarm system connected, via a switch (17), to the output of the pulse generator (14).

10. An apparatus according to claim 8, **characterized** in that the passive electrode (13) is made in the form of an envelope of an adjustable diameter which can be put on any finger.

*FIG.1*

*FIG.2*

FIG. 3

FIG. 4

# INTERNATIONAL SEARCH REPORT

International Application No PCT/SU 90/00177

| I. CLASSIFICATION OF SUBJECT MATTER (if several classification symbols apply, indicate all) 6 |
|---|

According to International Patent Classification (IPC) or to both National Classification and IPC

Int.Cl.$^5$   A 61 H 39/00; A 61 N 5/06

## II. FIELDS SEARCHED

### Minimum Documentation Searched 7

| Classification System | Classification Symbols |
|---|---|

Int.Cl.$^5$   A 61 H 39/00, 39/02, 39/06; A 61 N 5/06

### Documentation Searched other than Minimum Documentation to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | SU, A1, 1011124 (Tsentralny nauchno-issledovatelsky institut reflexoterapii), 15 April 1983 (15.04.83), fig.1,2, column 1, paragraph 5, column 2, paragraphs 3-6 and the claims | 5,7 |
| A | DE, A1, 3237398 (ČESKOSLOVENSKÁ AKADEMIA VĚD) 28 April 1983 (28.04.83), page 7, paragraph 3, fig. 4 | 1,5 |
| A | DE, A1, 2740969 (CASPERS, KARL HEINZ), 22 March 1979 (22.03.79), pages 7-8, fig. 1 | 1,7 |
| A | DE, A1, 2806582 (MESSERSCHMITT-BÖLKOW-BLOHM GMBH) 23 August 1979 (23.08.79), claims 1 and 8 | 5 |
| A | FR, A1, 2458279 (SKOVAJSA JOSEPH), 2 January 1981 (02.01.81), page 2, paragraphs 5,6,7, page 3, fig. 2 | 1,4,7 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 16 October 1990 (16.10.90) | 12 November 1990 (12.11.90) |

| International Searching Authority | Signature of Authorized Officer |
|---|---|
| ISA/SU | |

Form PCT/ISA/210 (second sheet) (January 1985)